# FASCICULE DE BREVET EUROPEEN

(11) **EP 2 826 539 B1**
(45) Date de publication et mention de la délivrance du brevet: **27.04.2022**
(21) Numéro de dépôt: 14174618.0
(22) Date de dépôt: 26.06.2014
(51) Int. Cl.: B01D 53/02, B01J 20/32, B01D 53/72, B01D 53/82, B01D 53/04, B01J 20/18, B01J 20/20, B01J 20/28

(54) **Cartouche filtrante pour appareil de purification d'air**
Filterpatrone für Luftreinigungsgerät
Filter cartridge for air-purification apparatus

(30) Priorité: 28.06.2013 FR 1356322
(43) Date de publication de la demande: 21.01.2015
(73) Titulaire: SEB S.A., 69130 Ecully (FR); Ethera, 38000 Grenoble (FR)
(72) Inventeur: Soulie, Jean-Pierre, 27200 VERNON (FR); Marchal, Eric, 27120 HOULBEC-COCHEREL (FR); Bigay, Yves, 38330 SAINT-ISMIER (FR); Chevallier, Emmanuel, 91190 GIF-SUR-YVETTE (FR)
(74) Mandataire: SEB Développement

(56) Documents cités:
- WO-A2-2010/027868
- DE-A1- 10 155 879
- FR-A1- 2 890 745
- JP-A- 2010 022 979
- US-A- 5 522 808

## Description

La présente invention concerne de manière générale le domaine des appareils purificateurs d'air et plus particulièrement des cartouches filtrantes pour ce type d'appareils et ayant des capacités d'absorption notamment des aldéhydes et formaldéhydes.

Par aldéhyde, on désigne toute molécule organique ayant une fonction carbonyle terminale choisie de préférence parmi le formaldéhyde, l'acétaldéhyde, le propionaldéhyde, le butyraldéhyde, l'acroléine, le pentanal, l'hexanal et le benzaldéhyde.

Les aldéhydes comptent parmi les polluants chimiques domestiques les plus abondants. Leurs sources sont extrêmement nombreuses. Elles peuvent être notamment en relation avec une production extérieure comme la photo-oxydation du méthane. Cependant, les sources principales d'émission des aldéhydes se trouvent à l'intérieur des habitations et sont très diverses : les résines et les colles servant à fabriquer les bois agglomérés, les panneaux de particules et les contreplaqués, les mousses isolantes urée-formol utilisées comme isolant thermique, par injection dans les murs et les cloisons, dans les revêtements textiles, papiers peints, peintures, cuirs...

Le formaldéhyde est également un agent conservateur, désinfectant et déshydratant. Pour ces raisons, il est abondamment employé comme solvant en milieu hospitalier pour la désinfection des instruments chirurgicaux mais aussi dans l'industrie des services funéraires où l'on pratique la thanatopraxie.

Compte tenu des effets nocifs de tels polluants chimiques sur la santé publique, il apparaît nécessaire d'assurer la purification de l'air ambiant des bâtiments d'habitation en réduisant la teneur en aldéhydes, et notamment en formaldéhyde, et offrant de nouveaux dispositifs de dépollution.

Selon l'art antérieur connu, les méthodes d'épuration des polluants chimiques gazeux présents dans l'air peuvent se classer en 2 catégories :
- Destruction du polluant par dégradation des composés organiques jusqu'à minéralisation totale c'est-à-dire jusqu'à les transformer en CO2 et H2O par oxydation ou photo-oxydation.
- Piégeage par des matériaux absorbants poreux qui retiennent les polluants, mais ne les dégradent pas. Ces matériaux sont de type zéolite ou charbon actif et sont couramment utilisés dans le traitement de l'air pour piéger les composés organiques volatils et les odeurs. Par exemple, on peut citer le document JP 2010022979 décrivant un matériau pour la filtration de mauvaise odeur dans un gaz. Le matériau comprend un support d'un composé de silicium organique ayant un groupe amino et / ou un groupe imino attaché au support et « un gonflement à l'eau minéral en couches ». Le document FR2890745 décrit un procédé de détection et/ou de dosage et/ou de piégeage d'au moins un aldéhyde, tel le formaldéhyde, comprenant une étape de mise en contact d'un flux gazeux avec une matrice nanoporeuse sol-gel d'oxydes métalliques, contenant au moins une molécule sonde portant au moins une fonction réactive pouvant réagir avec une fonction aldéhyde. Le document US5522808 décrit un dispositif et procédé de filtration de composants présents dans de la fumée chirurgicale. Le dispositif de filtration est composé d'éléments qui réagissent avec un ou plusieurs composants d'une fumée chirurgicale, telle que celle résultant de la galvanocautérisation et de la chirurgie au laser. Le dispositif de filtration, en particulier en conjonction avec un filtre séparé pour particules, enlève de la fumée chirurgicale le cyanure, le formaldéhyde, le benzène et la matière particulaire, ainsi que les substances responsables d'odeurs et de l'humidité.

La première catégorie est réalisée à partir de dispositifs utilisant des oxydants tels que l'ozone, ou favorisant l'oxydation comme les plasmas ou la photo-catalyse.

La deuxième catégorie utilise les pouvoirs et les capacités absorbants des matériaux poreux à grande surface spécifique (> 100m2/g), elle ne permet pas de dégrader les molécules, elle les retient sur un media poreux.

La première catégorie présente les désavantages d'être complexe et relativement coûteuse. De plus, elle peut générer des produits de sous décomposition qui peuvent s'avérer plus dangereux que le composé éliminé.

La deuxième catégorie présente l'inconvénient d'avoir des taux de piégeage très différents d'un composé chimique à éliminer à un autre. Ainsi par exemple le charbon actif est assez efficace pour absorber les composés aromatiques mais très inefficace pour absorber les aldéhydes et en particulier le formaldéhyde.

Les fabricants de matériaux absorbants essayent d'améliorer le pouvoir absorbant de leurs matériaux en les fonctionnalisant. Malheureusement, cette fonctionnalisation se fait par imprégnation qui a le désavantage de boucher les pores et donc de limiter les capacités de piégeage si l'on souhaite en mettre une quantité suffisante.

Le but de l'invention est d'améliorer les performances des cartouches filtrantes pour appareil de purification de l'air en y ajoutant un média absorbant de composés chimiques polluant de l'air envers des substances pour lesquels les matériaux absorbants actuels n'ont pas d'efficacité en particulier vis-à-vis des aldéhydes et notamment du formaldéhyde. La cartouche filtrante ainsi constituée assure le piégeage définitif et en grande quantité de la grande majorité de tous les polluants atmosphériques.

Ce but est atteint par l'intermédiaire d'une cartouche filtrante pour appareil de purification de l'air comprenant une structure assurant le maintien d'un média filtrant, le média filtrant comprenant un matériau absorbant classique choisi parmi le charbon actif ou la zéolite, caractérisée en ce que le média filtrant comprend en outre un matériau absorbant spécifique nanoporeux fonctionnalisé avec des molécules sondes de manière à permettre de piéger des polluants chimiques du type aldéhydes .

Le matériau absorbant spécifique est fabriqué par sol-gel pour incorporation in situ dans une structure nanoporeuse d'oxydes métalliques des molécules sondes apte à piéger des aldéhydes.

La molécule sonde portant une fonction réactive pouvant réagir avec une fonction aldéhyde est choisie parmi les énaminones et les couples β-dicétone/amine correspondants, les imines et les hydrazines, ou les sels dérivés de ces composés.

Selon une autre variante de réalisation, la structure assurant le maintien du média filtrant est une structure rigide alvéolaire, les alvéoles contenant le médiat filtrant.

Selon une autre variante de réalisation, un film micro-perforé est assemblé sur les faces amont et aval de la structure rigide alvéolaire.

Selon une autre variante de réalisation, le taux de remplissage des alvéoles au média filtrant est supérieur à 40%.

Le matériau absorbant de l'invention est sous forme de granulé cylindrique ou broyé dont les dimensions sont comprises entre 0.8 et 2mm.

Selon une autre variante de réalisation, la forme des granulés est cylindrique avec un rapport L/D>1 dans lequel L correspond à la longueur d'un granulé et D correspond au diamètre d'un granulé.

Selon une autre variante de réalisation, la structure assurant le maintien du média filtrant est un assemblage de plusieurs films sur lesquels sont imprégnés/saupoudrés le matériau absorbant classique et le matériau absorbant spécifique.

Selon une autre variante de réalisation, la masse de matériau absorbant spécifique représente entre 5 et 95% de la masse de matériau absorbant classique.

Selon une autre variante de réalisation, la surface spécifique du matériau absorbant spécifique est comprise entre 600 et 1200m²g.

L'invention concerne également un appareil de purification de l'air comprenant une cartouche filtrante telle que définie ci-dessus.

D'autres caractéristiques et avantages de la présente invention apparaîtront plus clairement à la lecture de la description détaillée qui suit de modes de réalisation de l'invention donnés à titre d'exemple nullement limitatifs et illustrés par les dessins annexés, dans lesquels :
- la figure 1 représente schématiquement une première structure de cartouche filtrante selon l'invention ;
- les figures 2A et 2B représentent respectivement une vue de face et une vue en perspective d'une deuxième structure de cartouche filtrante selon l'invention ;
- la figure 3 représente l'évolution de la concentration de formaldéhyde dans une pièce avec une première variante de réalisation de la cartouche selon l'invention ;
- la figure 4 représente l'évolution de la concentration de formaldéhyde dans une pièce avec une deuxième variante de réalisation de la cartouche selon l'invention.

L'invention consiste essentiellement en une cartouche filtrante comprenant un média filtrant. Le média filtrant comprend un ou plusieurs lits de matériaux adsorbants, combinés ou séparés, permettant d'éliminer par absorption les composés organiques volatils présents dans l'air intérieur. Selon l'invention, les composés organiques volatils absorbés sont notamment des aldéhydes et/ou des solvants. La cartouche filtrante est destinée à être introduite dans un appareil de purification de l'air ambiant pouvant fonctionner à des débits de quelques m3/h à plusieurs milliers de m3/h.

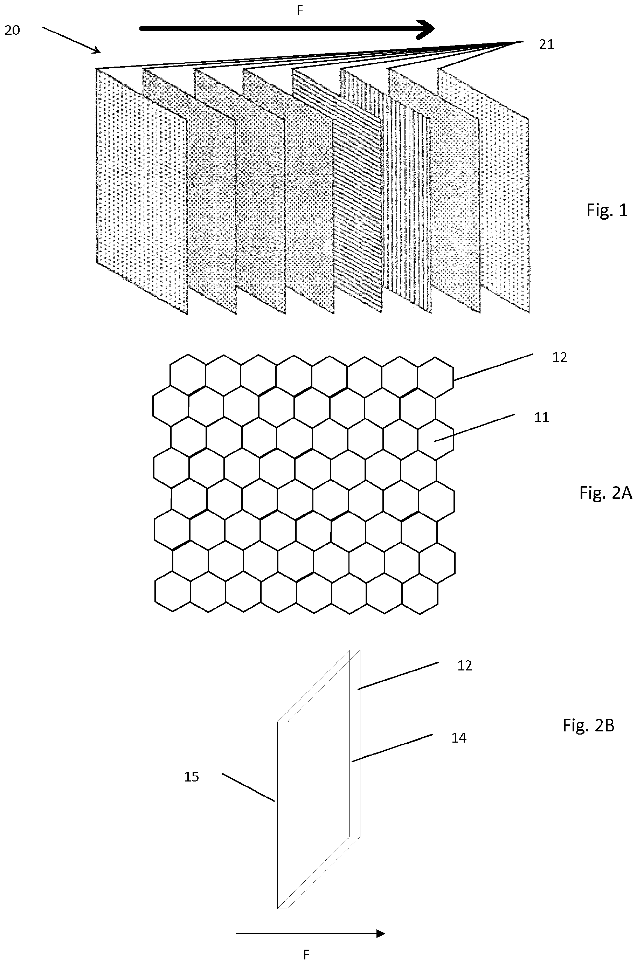

Les figures 1, 2A et 2B représentent des exemples de structures de cartouches filtrantes susceptibles d'être utilisées dans le cadre de l'invention.

Ces structures permettent de maintenir le média filtrant selon l'invention dans le flux d'air traversant l'appareil de purification de l'air.

La figure 1 représente une structure 20 (connue en soi) comprenant une succession de films 21. Dans cette variante, le média filtrant est imprégné dans un ou plusieurs films 21. L'ensemble des films est ensuite assemblé (par exemple par collage ou soudage) pour former la cartouche filtrante.

Les dimensions et les nombres de films 21 dépendent essentiellement des performances souhaitées de l'appareil de purification de l'air.

Selon les figures 2A et 2B, la cartouche filtrante comprend une structure 12 rigide alvéolaire. Selon cette variante de réalisation, la structure est de type nid d'abeille. Le média filtrant est placé dans les alvéoles 11 de la structure 12. Afin de garantir le maintien du média filtrant dans les alvéoles 11, un film micro-perforé est placé sur les deux faces aval 14 et amont 15 de la structure 12 rigide alvéolaire. Le film est par exemple totalement transparent vis-à-vis du flux d'air et ne possède pas de fonction de filtration.

Pour les deux variantes de structures de cartouches filtrantes, la cartouche est placée dans l'appareil de purification de l'air perpendiculairement au flux d'air F.

La géométrie de la cartouche filtrante selon l'invention peut être sous différentes formes, plane ou en volume. La cartouche filtrante contient un mélange et/ou des couches successives de granulés contenant un ou plusieurs absorbants.

Selon l'invention, le média filtrant comprend une association d'au moins un matériau absorbant classique pouvant éliminer un large spectre de composés organiques volatils de l'air intérieur des habitations et un matériau absorbant spécifique pour piéger des composés très mal éliminés par le matériau absorbant classique comme par exemple les composés de type aldéhydes et plus particulièrement les formaldéhydes.

Selon l'invention, le matériau absorbant classique est choisi parmi le charbon actif ou les zéolites.

Le matériau absorbant spécifique est un matériau apte à piéger spécifiquement un polluant ou une famille de polluant. Par exemple le média filtrant peut piéger la famille des aldéhydes par l'intermédiaire de molécules sondes (ou principe actif) adaptées et incorporées dans une structure nanoporeuse. Un choix éclairé de molécules sondes peut permettre de piéger plus spécifiquement un polluant comme par exemple le formaldéhyde. Le procédé de fabrication du matériau absorbant spécifique de type sol-gel permet une introduction du principe actif lors de son élaboration (in situ) et non pas par imprégnation. Le procédé de fabrication du matériau absorbant spécifique permet d'introduire beaucoup de principe actif sans boucher les pores puisqu'il n'y a pas d'imprégnation. Le bouchage des pores par imprégnation est ainsi évité.

Ainsi en choisissant de façon appropriée la molécule sonde, il est possible éliminer les polluants nocifs tels que le formaldéhyde, que les matériaux absorbants classiques ne peuvent pas ou peu éliminer.

Dans le cas du matériau absorbant spécifique sol-gel, le polluant réagit avec le principe réactif pour donner un troisième corps d'un poids moléculaire plus important et moins nocif qui restera piégé dans le réseau nanoporeux du matériau absorbant spécifique. Contrairement aux autres absorbants, ce matériau absorbant spécifique effectue un piégeage définitif.

A titre d'exemple, le matériau absorbant spécifique est choisi parmi les matériaux décrits dans la demande de brevet FR2890745.

Notamment et de manière non limitative, le matériau comprend une matrice nanoporeuse sol-gel d'oxydes métalliques, ladite matrice contenant au moins une molécule sonde portant au moins une fonction réactive pouvant réagir avec une fonction aldéhyde.

La molécule sonde portant une fonction réactive pouvant réagir avec une fonction aldéhyde est choisie parmi les énaminones et les couples β-dicétone/amine correspondants, les imines et les hydrazines, ou les sels dérivés de ces composés.

Les énaminones répondent à la formule ci-dessous : 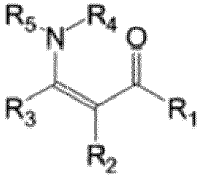 dans laquelle :
- R1 correspond à un hydrogène, un radical alkyle ou aryle,
- R2 correspond à un hydrogène,
- R3 correspond à un hydrogène, un radical alkyle ou aryle,
- R4 correspond à un hydrogène, un radical alkyle ou aryle,
- R5 correspond à un hydrogène.

Par ailleurs, le couple β-dicétone/amine répond à la formule ci-dessous : 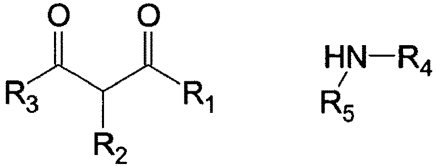 dans laquelle :
- R1 correspond à un hydrogène, radical alkyle ou aryle,
- R2 correspond à un hydrogène,
- R3 correspond à un hydrogène, un radical alkyle ou aryle,
- R4 correspond à un hydrogène, un radical alkyle,
- R5 correspond à un hydrogène, ou un sel correspondant.

Par ailleurs, l'imine est une base de Schiff choisie parmi le jaune d'acridine, le jaune de méthyle ou de diméthyle.

L'hydrazine répond à la formule suivante : 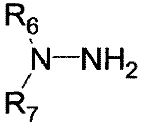 dans laquelle :
- R6 correspond à un hydrogène, un radical alkyle en C1-C20, préférentiellement en C1-C10, plus préférentiellement méthyle, éthyle, isopropyle, butyle, isobutyle, tert-butyle et pentyle, un radical aryle en C3-C16, notamment phényle et aryle sulfonyle,
- R7 correspond à un radical aryle en C3-C16, notamment phényle et aryle sulfonyle.

Selon l'invention, la matrice nanoporeuse sol-gel d'oxydes métalliques est élaborée à partir d'au moins un oxyde métallique de formule ci-dessous :
M(X)m(OR8)n(R9)p
dans laquelle :
   - M correspond à un métal choisi parmi le silicium, l'aluminium, le titane, le zirconium, le niobium, le vanadium, l'yttrium et le cérium,
   - R8 et R9 correspondent indépendamment à un radical alkyle ou aryle,
   - n, m et p sont des entiers, tels que leur somme soit égale à la valence de M et que n soit supérieur ou égal à 2,
   - X est un halogène.

Le matériau absorbant spécifique tel que défini ci-dessus, permettant de piéger spécifiquement les aldéhydes et notamment le formaldéhyde, possède une efficacité de capacité de piégeage vis-à-vis du formaldéhyde au moins 100 fois supérieure à celle du charbon actif dédié aux composés organiques volatils totaux et 10 fois supérieure à celle du charbon actif imprégné spécifiquement pour le piégeage du formaldéhyde gazeux. La capacité de piégeage définitif du matériau absorbant spécifique est au minimum 0,01g de formaldéhyde par gramme de matériau. Par ailleurs, même une fois saturé, ce matériau absorbant spécifique a une capacité de piégeage par adsorption équivalente au charbon actif imprégné spécifiquement pour le piégeage du formaldéhyde gazeux.

Selon l'art antérieur, le charbon actif ou les zéolites éliminent très mal les aldéhydes et en particulier le formaldéhyde alors que ce gaz est d'une part très présent dans l'air intérieur et d'autre part nocif sur la santé. Ainsi, en associant un matériau absorbant classique et un matériau absorbant spécifique, particulièrement efficace pour les aldéhydes et notamment les formaldéhydes, la cartouche filtrante selon l'invention permet d'éliminer à la fois les aldéhydes et en particulier le formaldéhyde grâce à la présence du matériau absorbant spécifique nanoporeux fonctionnalisé avec des molécules sondes et les autres composés organiques volatils en particulier la famille des hydrocarbures aromatiques monocycliques (Benzène, Toluène, Ethylène, Xylène,...) grâce au charbon actif ou aux zéolites.

L'association des matériaux absorbants classiques et spécifiques est constituée soit par mélange des matériaux ou par association en couches successives mono-matériau.

Les matériaux absorbants classiques et spécifiques peuvent être pré-mélangés afin d'être introduits de manière homogène dans le filtre ou bien être introduits séparément par saupoudrage sur plusieurs étages ou sur différents niveaux séparés physiquement.

Selon l'invention, les performances de dépollution/filtration de la cartouche sont déterminées notamment par les paramètres suivants :
- la proportion en masse entre le matériau absorbant classique et le matériau absorbant spécifique,
- la surface spécifique du matériau absorbant spécifique,
- la forme du matériau absorbant spécifique,
- les caractéristiques physiques et chimiques du matériau absorbant classique.

La proportion en masse entre le matériau absorbant classique et le matériau absorbant spécifique peut varier d'un facteur 95/5 à 5/95 : le choix particulier est réalisé en fonction de la nature et de la quantité des polluants présents dans l'air intérieur et des performances souhaitées à atteindre.

A titre d'exemple pour un air contenant 20µg/m3 de formaldéhyde et 200µg/m3 d'autres composés organiques volatils, il sera utilisé préférablement un rapport massique matériau absorbant spécifique/charbon actif de 10/90. Toutefois, il est possible de doubler, quadrupler, etc l'efficacité du média filtrant selon l'invention en doublant, quadruplant, etc. la quantité de matériau absorbant spécifique.

Ainsi dans le cas d'air très pollué en formaldéhyde contenant par exemple 10 fois plus de formaldéhyde que d'autres composés organiques volatils, il sera utilisé un filtre ayant un rapport de matériau absorbant spécifique/charbon actif de 90/10.

La surface spécifique recherchée pour le matériau absorbant spécifique est inversement proportionnelle à la taille des pores. Ainsi, plus la surface spécifique est grande plus la taille des pores est petite. Ainsi pour avoir une surface spécifique de l'ordre de 1000m2/g, le diamètre des pores est typiquement aux alentours de l'ordre du nanomètre.

Il est donc recherché le meilleur compromis entre une surface spécifique maximum qui permet d'accroître la capacité et l'efficacité de piégeage et une limite à ne pas dépasser afin d'avoir des pores de taille suffisante pour que les polluants puissent entrer dans les pores. La plage de surface spécifique visée se situe entre 100 et 1500m2/g dépendant de la taille de la molécule polluante à éliminer. Par exemple pour le formaldéhyde, on préfèrera se situer entre 600 et 1200m²/g.

Le choix de la forme de granulé pour le matériau absorbant spécifique est déterminé pour avoir le maximum de surface de léchage (surface externe du granulé) pour accroître l'efficacité de piégeage tout en ayant la plus faible perte de charge pour minimiser l'énergie nécessaire à la dépollution.

Par exemple dans le cas de granulé cylindrique, on sait que la surface de léchage est proportionnelle à l'inverse du diamètre moyen des granulés. Ainsi la diminution du diamètre de granulé augmente les performances grâce à une augmentation de la surface de piégeage. Par contre, cette diminution entraîne un accroissement des pertes de charges et de l'énergie nécessaire. En effet, le lit de granulé devient alors plus compact et donc moins transparent au flux d'air le traversant. Typiquement des granulés de l'ordre du millimètre (0,2 à 8mm) présentent le meilleur compromis entre la meilleure efficacité et une perte de charge pas trop importante.

Les formes cylindriques de forme allongée, c'est-à-dire ayant un rapport L/D >1 (L longueur, D diamètre), sont très intéressantes car elles sont facilement fabricables par moulage ou extrusion. Leurs formes allongées évitent que les surfaces planes s'accolent pour ne pas diminuer la surface de léchage et ne pas augmenter de pertes de charges et de l'énergie. Dans ce cas, les dimensions (longueur) de l'ordre du millimètre (0,2 à 8mm) conduisent également au meilleur compromis.

Selon un autre mode de réalisation, le granulé peut prendre la forme broyée conduisant à des particules de l'ordre du millimètre (0,2 à 8mm). Cette forme présente également un intérêt car elle donne pour un diamètre de particule sensiblement identique une surface de léchage supérieure au cas des particules sphériques.

Des granulés de taille inférieure au millimètre peuvent être également utilisés à condition d'utiliser des supports (fibre) inter-granulés permettant de disperser le granulé pour diminuer la perte de charge. Il peut dans ce cas s'envisager des films de granulé mixte matériau absorbant classique/ matériau absorbant spécifique emprisonnés dans des tissus permettant d'augmenter la surface du filtre afin de diminuer les pertes de charges.

Par ailleurs, d'autres critères tels que les dimensions de la structure rigide alvéolaire et le taux de remplissage des alvéoles peuvent également être ajustés pour obtenir les performances souhaitées de la cartouche filtrante selon l'invention. Ainsi, la taille des alvéoles de la structure est comprise entre 1 et 10 fois la taille maximale d'un granulé de matériau absorbant spécifique. Cette dimension permet d'obtenir une bonne répartition des granulés dans les alvéoles et de pourvoir recevoir au minimum 1 granulé par alvéole de la structure rigide alvéolaire.

De même, le taux de remplissage des alvéoles en mélange de matériaux absorbant classique et spécifique est par exemple d'au moins 40%. Ceci permet de limiter l'encombrement de la cartouche filtrante selon l'invention.

La cartouche filtrante selon l'invention ne remet pas en cause l'architecture générale des appareils de purification de l'air connue. En effet, il suffit d'insérer la cartouche filtrante selon l'invention en lieu et place de la cartouche de l'art antérieur. La composition et les caractéristiques de la cartouche filtrante selon l'invention seront alors ajustées en fonction des caractéristiques de l'appareil de purification de l'air, notamment en fonction du débit d'air et des dimensions de la cartouche.

Exemples de dimensionnement de cartouche filtrante selon l'invention :
Le premier exemple est défini pour réaliser la dépollution d'une pièce ayant les caractéristiques suivantes :
   - volume : 12m³,
   - renouvellement d'air neuf : 5m³/h,
   - taux d'émission de formaldéhyde : 70,6µg/m³.
Les caractéristiques de l'appareil de purification utilisant la cartouche filtrante selon l'invention sont les suivantes :
   - débit d'air : 140m³/h,
   - cartouche filtrante alvéolaire (nid d'abeille) : largeur 21cm, longueur 53cm, profondeur 1cm (soit une surface totale de passage de 0,068m2),
   - masse de matériau absorbant spécifique : 20g.

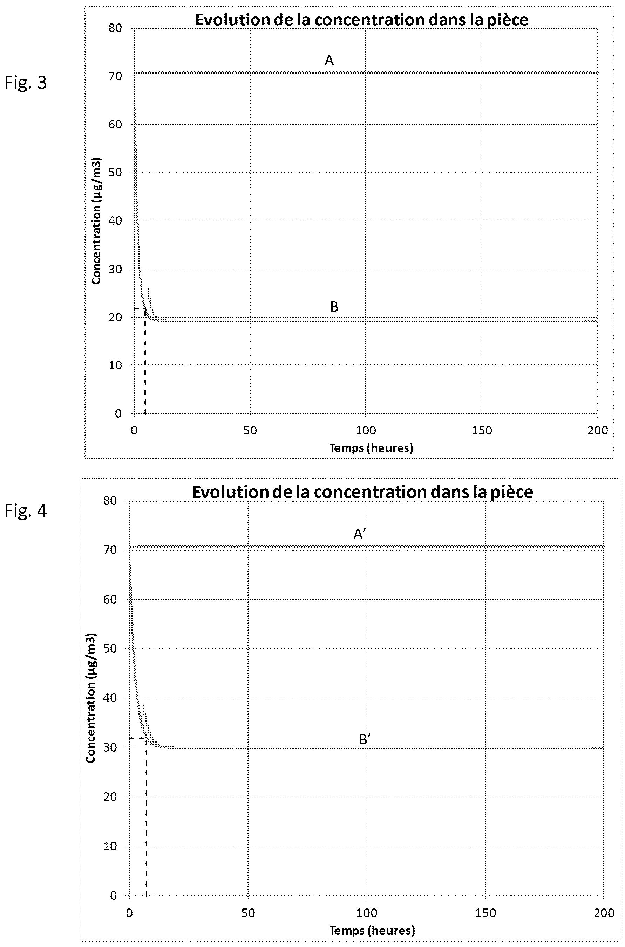

En figure 3 est représentée l'évolution de la concentration de formaldéhyde dans la pièce.

La courbe A représente la valeur témoin à 70,6µg/m3 sans appareil de purification de l'air selon l'invention.

La courbe B représente la variation de la concentration lorsque l'appareil de l'air selon l'invention est en fonctionnement.

Ainsi, on note qu'en quelques heures de fonctionnement le taux d'émission de formaldéhydes descend sous 20µg/m3 (inférieur au taux de 30µg/m3 recommandé dans les établissements recevant du public).

Le deuxième exemple est défini pour réaliser la dépollution d'une pièce ayant les mêmes caractéristiques que dans l'exemple précédent mais avec les caractéristiques de l'appareil de purification d'air utilisant la cartouche filtrante selon l'invention qui sont les suivantes :
- débit d'air : 70m³/h,
- cartouche filtrante alvéolaire (nid d'abeille) : largeur 24cm, longueur 29cm, profondeur 1cm (soit une surface totale de passage de 0,042m2),
- masse de matériau absorbant spécifique : 10g.

En figure 4 est représentée l'évolution de la concentration de formaldéhyde dans la pièce.

La courbe A' représente la valeur témoin à 70,6µg/m³ sans appareil de purification de l'air selon l'invention.

La courbe B' représente la variation de la concentration lorsque l'appareil de l'air selon l'invention est en fonctionnement.

Ainsi, on note qu'en quelques heures de fonctionnement, le taux d'émission de formaldéhydes est de l'ordre de 30µg/m³.

On comprendra que diverses modifications et/ou améliorations évidentes pour l'homme du métier peuvent être apportées aux modes de réalisation de l'invention décrits dans la présente description sans sortir du cadre de l'invention défini par les revendications annexées.

Ainsi, les deux exemples de réalisation ont été donnés pour un appareil de purification de l'air à usage domestique (pièce de quelques m3). Des applications des cartouches filtrantes selon l'invention dans un appareil de purification d'air de plus grandes dimensions (par exemple adapté pour un établissement recevant du public) sont envisageables. Il suffit soit d'utiliser plusieurs cartouches soit des cartouches de plus grandes dimensions.

## Revendications

1. Cartouche filtrante pour appareil de purification de l'air comprenant une structure (12, 20) assurant le maintien d'un média filtrant,
le média filtrant comprenant un matériau choisi parmi le charbon actif ou la zéolite,
**caractérisée en ce que** le média filtrant comprend en outre un matériau nanoporeux qui est :
- fonctionnalisé avec des molécules sondes aptes à piéger des aldéhydes qui sont choisies parmi les énaminones et les couples β-dicétone/amine correspondants, les imines et les hydrazines, ou les sels dérivés de ces composés,
- fabriqué par sol-gel pour incorporation in situ dans une structure nanoporeuse d'oxydes métalliques desdites molécules sondes,
- sous forme de granulé cylindrique ou broyé dont les dimensions sont comprises entre 0.2 et 8 mm.

2. Cartouche filtrante pour appareil de purification de l'air selon la revendication 1, **caractérisée en ce que** la structure assurant le maintien du média filtrant est une structure (12) rigide alvéolaire, les alvéoles (11) contenant le médiat filtrant.

3. Cartouche filtrante pour appareil de purification de l'air selon la revendication précédente, **caractérisée en ce qu'**un film micro-perforé est assemblé sur les faces amont (15) et aval (14) de la structure (12) rigide alvéolaire.

4. Cartouche filtrante pour appareil de purification de l'air selon la revendication 2, **caractérisée en ce que** le taux de remplissage des alvéoles au média filtrant est supérieur à 40%.

5. Cartouche filtrante pour appareil de purification de l'air selon l'une quelconque des revendications précédentes, **caractérisée en ce que** la forme des granulés est cylindrique avec un rapport L/D>1 dans lequel L correspond à la longueur d'un granulé et D correspond au diamètre d'un granulé.

6. Cartouche filtrante pour appareil de purification de l'air selon la revendication 1, **caractérisée en ce que** la structure (20) assurant le maintien du média filtrant est un assemblage de plusieurs films (21) sur lesquels sont imprégnés/saupoudrés le matériau absorbant classique et le matériau absorbant spécifique.

7. Cartouche filtrante pour appareil de purification de l'air selon l'une des revendications précédentes, **caractérisée en ce que** la masse de matériau absorbant spécifique représente entre 5 et 95% de la masse de matériau absorbant classique.

8. Cartouche filtrante pour appareil de purification de l'air selon l'une des revendications précédentes, **caractérisée en ce que** la surface spécifique du matériau absorbant spécifique est comprise entre 600 et 1200m²g.

9. Appareil de purification de l'air, **caractérisé en ce qu'**il comprend au moins une cartouche filtrante définie selon l'une des revendications 1 à 8.

## Patentansprüche

1. Filtereinsatz für eine Luftreinigungseinrichtung, die eine Struktur (12, 20) umfasst, die den Halt eines Filtermediums gewährleistet,
wobei das Filtermedium ein Material umfasst, das aus Aktivkohle oder Zeolith ausgewählt ist,
**dadurch gekennzeichnet, dass** das Filtermedium ferner ein nanoporöses Material umfasst, das:
- funktionalisiert ist mit Sondenmolekülen, die geeignet sind, Aldehyde einzufangen, die unter den Enaminonen und den entsprechenden β-Diketon/Amin-Paaren, den Iminen und den Hyrazinen oder den von diesen Verbindungen abgeleiteten Salzen gewählt sind,
- hergestellt ist nach dem Sol-Gel-Verfahren für den in-situ-Einbau in eine nanoporöse Struktur aus Metalloxyden besagter Sondenmoleküle,
- in Form eines zylindrischen oder gemahlenen Granulats vorliegt, dessen Abmessungen zwischen 0,2 und 8 mm liegen.

2. Filtereinsatz für eine Luftreinigungseinrichtung nach Anspruch 1, **dadurch gekennzeichnet, dass** die Struktur, die den Halt des Filtermediums gewährleistet, eine starre wabenförmige Struktur (12) ist, wobei die Waben (11) das Filtermedium enthalten.

3. Filtereinsatz für eine Luftreinigungseinrichtung nach dem vorstehenden Anspruch, **dadurch gekennzeichnet, dass** eine mikroperforierte Folie an den stromaufwärts liegenden (15) und stromabwärts liegenden (14) Seiten der starren wabenförmigen Struktur (12) angeordnet ist.

4. Filtereinsatz für eine Luftreinigungseinrichtung nach Anspruch 2, **dadurch gekennzeichnet, dass** der Füllungsgrad der Waben mit Filtermedium mehr als 40 % beträgt.

5. Filtereinsatz für eine Luftreinigungseinrichtung nach einem vorstehenden Ansprüche, **dadurch gekennzeichnet, dass** die Form der Granulate zylindrisch mit einem Verhältnis L/D>1 ist, worin L der Länge eines Granulats und D dem Durchmesser eines Granulats entspricht.

6. Filtereinsatz für eine Luftreinigungseinrichtung nach Anspruch 1, **dadurch gekennzeichnet, dass** die Struktur (20), die den Halt des Filtermediums gewährleistet, eine Anordnung mehrerer Folien (21) ist, auf denen das klassische absorbierende Material und das spezifische absorbierende Material imprägniert/aufgestäubt ist.

7. Filtereinsatz für eine Luftreinigungseinrichtung nach einem der vorstehenden Ansprüche, **dadurch gekennzeichnet, dass** die Masse des spezifischen absorbierenden Materials für zwischen 5 und 95 % der Masse des klassischen absorbierenden Materials steht.

8. Filtereinsatz für eine Luftreinigungseinrichtung nach einem der vorstehenden Ansprüche, **dadurch gekennzeichnet, dass** die spezifische Oberfläche des spezifischen absorbierenden Materials zwischen 600 und 1200 m²g liegt.

9. Luftreinigungseinrichtung, **dadurch gekennzeichnet, dass** sie zumindest einen nach einem der Ansprüche 1 bis 8 definierten Filtereinsatz umfasst.

## Claims

1. Filter cartridge for air-purification apparatus comprising a structure (12, 20) ensuring the holding of a filter medium,
the filter medium comprising a material chosen from among active carbon or zeolite,
**characterised in that** the filter medium further comprises a nanoporous material which is:
- functionalised with probe molecules capable of trapping aldehydes which are chosen from among enaminones and corresponding β-dicetone/amine couples, imines and hydrazines, or salts derived from these compounds,
- fabricated by sol-gel for in situ incorporation in a nanoporous structure of metal oxides of said probe molecules,
- in the form of cylindrical granulate or crushed, the dimensions of which are between 0.2 and 8mm.

2. Filter cartridge for air-purification apparatus according to claim 1, **characterised in that** the structure ensuring the holding of the filter medium is a rigid honeycomb structure (12), the holes (11) containing the filter medium.

3. Filter cartridge for air-purification apparatus according to the preceding claim, **characterised in that** a microperforated film is assembled on the upstream (15) and downstream (14) faces of the rigid honeycomb structure (12).

4. Filter cartridge for air-purification apparatus according to claim 2, **characterised in that** the rate of filling holes with the filter medium is greater than 40%.

5. Filter cartridge for air-purification apparatus according to any one of the preceding claims, **characterised in that** the shape of the granulates is cylindrical with a ratio L/D>1 wherein L corresponds to the length of a granulate and D corresponds to the diameter of a granulate.

6. Filter cartridge for air-purification apparatus according to claim 1, **characterised in that** the structure (20) ensuring the holding of the filter medium is an assembly of several films (21) on which the conventional absorbent material and the specific absorbent material are impregnated/sprinkled.

7. Filter cartridge for air-purification apparatus according to one of the preceding claims, **characterised in that** the specific absorbent material mass represents between 5 and 95% of the conventional absorbent material mass.

8. Filter cartridge for air-purification apparatus according to one of the preceding claims, **characterised in that** the specific surface of the specific absorbent material is between 600 and 1200m²g.

9. Air-purification apparatus, **characterised in that** it comprises at least one filter cartridge defined according to one of claims 1 to 8.
